# EUROPEAN PATENT APPLICATION

(11) **EP 2 062 912 A1**
(43) Date of publication of application: **27.05.2009**
(21) Application number: 07121523.0
(22) Date of filing: 26.11.2007
(51) Int. Cl.: C07K 1/14

(54) **Selective enrichment of post-translationally modified proteins and/or peptides**

(71) Applicant: Koninklijke Philips Electronics N.V., 5621 BA Eindhoven (NL)
(72) Inventor: The designation of the inventor has not yet been filed
(74) Representative: Plünnecke, Ingo

(57) **Abstract**

The present invention relates to the selective enrichment of post-translationally modified proteins and/or peptides from complex samples by combining a particular protein/peptide labeling protocol with the specific selection of the post-translationally modified proteins and/or peptides to be analyzed. In particular, the invention relates to methods for the separation and/or analysis of glycosylated proteins and/or peptides.

## Description

### SUBJECT OF THE INVENTION

The present invention relates to the selective enrichment of post-translationally modified proteins and/or peptides from complex samples by combining a particular protein/peptide labeling protocol with the specific selection of the post-translationally modified proteins and/or peptides to be analyzed. In particular, the invention relates to methods for the separation and/or analysis of glycosylated proteins and/or peptides.

### BACKGROUND OF THE INVENTION

The identification, separation, and analysis of particular proteins or subsets of proteins from complex samples are invaluable for unraveling how biological processes occur at a molecular level or to which degree proteins differ among various cell types or between physiological states.

A major challenge in modem biology is directed to the understanding of the expression, function, and regulation of the entire set of proteins encoded by an organism, a technical field commonly known as proteomics. However, as there is no possibility to amplify proteins, research in this field is generally rather tedious because even a cell extract of a relatively simple prokaryotic organism contains a multitude of proteins encompassing a huge range of concentrations. Therefore, such a task is beyond the capabilities of any current single analytical methods.

Thus, due to the methodological constraints proteome analysis relies not only to methods for identifying and quantifying proteins but - to a considerable extent - also on methods allowing their accurate and reliable separation according to their structural and/or functional properties, with these subsets being then better accessible to further analysis.

The proteome is of dynamic nature, with alterations in protein synthesis, activation, and/or post-translational modification in response to external stimuli or alterations in the cellular environment. Therefore, the proteome's inherent complexity exceeds that of the genome or the transcriptome the mRNA complement of a cell.

Due to the extraordinary amount of data to be processed in such proteomic studies the protein/peptide identification process demands tremendous resolving power. Two methods commonly used to resolve such highly complex mixtures are two-dimensional gel electrophoresis (2D-GE; cf., e.g., O'Farrell, P.H. (1975) J. Biol. Chem. 250, 4007-4021) and (two-dimensional) liquid chromatography ((2D)-LC; cf., e.g., Lipton, M.S. et al. (2002) Proc. Natl. Acad. Sci. USA 99, 11049-11054). The peptides and proteins isolated by 2D-GE or 2D-LC are usually identified by mass spectrometry or by determining amino acid composition and/or amino acid sequence.

However, although useful for many applications these identification techniques have major drawbacks with regard to proteomic studies, where highly complex samples are to be investigated. For example, hydrophobic membrane proteins, highly basic or acidic proteins, very large or very small proteins are often poorly resolved via 2D-GE. Furthermore, the detection (sensitivity) limits of these methods as well as shortcomings in labeling technology do not allow for the reliable analysis of multiple samples in parallel, e.g., for comparing relative protein levels between different disease groups, different progression stages of a disease, and between disease stages vs. healthy controls or for performing high-throughput screening analyses.

Therefore, it is highly desirable to develop methodologies which overcome the above limitations and enable processing of multiple complex samples in parallel.

Another important facet of protein analysis in general and proteomics in particular relates to the possibility to study post-translational protein modifications, which can affect activity and binding of a protein and alter its role within the cell (cf., e.g., Pandey, A. and Mann, M. (2000) Nature 405, 837-846). For example, the (reversible) phosphorylation of proteins is crucial for the regulation of many signal transduction cascades such as G-protein-coupled receptor signaling or phospho-tyrosine kinase signaling, protein glycosylation plays a predominant role in cell/cell- and cell/substrate-recognition in multicellular organisms, whereas an ubiquitination *inter alia* labels proteins for degradation.

One of the unique features of proteomics is that post-translational modifications can be investigated at a more global level, thus allowing the analysis of the entire subset of proteins comprising a particular modification. The expressed products of a single gene represent a protein population that may contain large amounts of micro-heterogeneity, each different state (i.e. analogous proteins differing in the number of post-translationally modified amino acid residues) adding a large amount of diversity to the expression profile of that protein.

Currently, there are several techniques available, for example mass spectrometry, which can in principle distinguish between analogous proteins or peptides due to the presence or absence of a specific modification. However, these changes are frequently not observed in global proteomic studies due to a limited sensitivity of detection. Thus, in order to study a specific post-translational modification, it would be helpful to enrich a sample for that modification, usually by some form of affinity purification, and/or to separate the enriched subset of modified proteins from that sample. However, the available methods are generally hampered by the requirement to label the proteins with appropriate affinity tags or the need to use specific antibodies or other reagents which might interfere with further analyses. Therefore, such methods based on affinity purification are particularly not suitable for processing multiple samples in parallel.

In addition, it is generally cumbersome to distinguish different subsets of proteins and/or peptides bearing a particular modification (e.g., tyrosine-phosphorylated *versus* serine/threonin-phosphorylated proteins or *N*-linked glyco-proteins *versus O*-linked glyco-proteins *versus* glycosylphosphatidylinisotol-anchored protein) based on capturing or affinity purification protocols.

The study of protein glycosylation has grown exponentially in recent years as researchers from various disciplines have come to realize that key cellular functions are regulated by this type of ubiquitous post-translational modification.

Importantly, glycan composition significantly reflects differences in cell types and states, e.g. species, tissues, developmental stages, etc. Additionally, glycans have much higher potential to exert structural diversity than nucleic acids and proteins (Laine, R.A. (1994) Glycobiology 4, 759-767). The number of saccharide components is relatively small including, e.g., glucose, *N*-acetyl glucosamine, mannose, galactose, *N*-acetyl galactosamine, L-fucose, L-xylose, L-arabinose, and *N*-acetyl neuraminic acid, but the high variation in linkage and branching makes glycosylation probably the most complex post-translational modification.

Furthermore, cellular glycosylation profiles were shown to change significantly during oncogenesis (reviewed, e.g., in Caprioli, R. M. (2005) Cancer Res. 65, 10642-10645); hence, the search continues for tumor-secreted glyco-proteins that can serve as biomarkers for tumor diagnostics.

Therefore, it is a continuing need for methods allowing the selective enrichment ofpost-translationally modified proteins and/or peptides from complex samples. In particular, it would be desirable to provide methods for the separation and/or discrimination of glycosylated proteins and/or peptides not only with high sensitivity but also without the requirement of specific reagents. Furthermore, it would also be desirable to provide methods that allow for performing multiplexed analyses.

### OBJECT AND SUMMARY OF THE INVENTION

It is an objective of the present invention to provide novel approaches for the selective enrichment of post-translationally modified peptides and/or proteins, in particular of glycosylated proteins and/or peptides, from complex samples. More specifically, it is an object of the present invention to provide methods for performing multiple such analyses in parallel.

It is a further objective of the present invention to provide methods that allow separating post-translationally modified proteins and/or peptides from their unmodified counterparts and/or to discriminate between different subsets of these modified proteins and/or peptides.

These objectives as well as others which will become apparent from the ensuing description are attained by the subject matter of the independent claims. Some of the preferred embodiments of the present invention are defined by the subject matter of the dependent claims.

In one embodiment, the present invention relates to a method for the selective enrichment and/or separation of post-translationally modified proteins and/or peptides from a sample, comprising:
(a) single or double chemical labeling of the proteins and/or peptides comprised in the sample;
(b) capturing a subset of post-translationally modified proteins and/or peptides comprising a specific post-translational modification to be analyzed; and
(c) separating the captured subset of post-translationally modified proteins and/or peptides.

In a preferred embodiment of the invention, the post-translational modification to be analyzed is glycosylation.

In a further embodiment, the method further comprises cleaving the proteins into peptides prior to and/or concomitantly with performing step (a).

In another preferred embodiment of the inventive method, the double chemical labeling comprises an isotopic and an isobaric labeling. Particularly preferably, the isotopic labeling is performed prior to the isobaric labeling.

In some embodiments, the isotopic labeling is performed prior to cleaving the proteins into peptides.

In case of the analysis of glycosylated proteins and/or peptides step (b) typically comprises at least one of lectin affinity capture and glycoprotein chemical capture.

In another preferred embodiment of the inventive method, step (c) comprises removing the post-translational modification from at least a first subset of the separated post-translationally modified proteins and/or peptides. Typically, the post-translational modification is removed chemically or enzymatically.

In another embodiment, the method further comprises analyzing the separated proteins and/or -peptides by means of mass spectrometry. In some embodiments, the method is performed in a high-throughput format.

The method of the present invention may be used for performing qualitative and/or quantitative proteomic analyses.

Other embodiments of the present invention will become apparent from the detailed description hereinafter.

### FIGURE LEGENDS

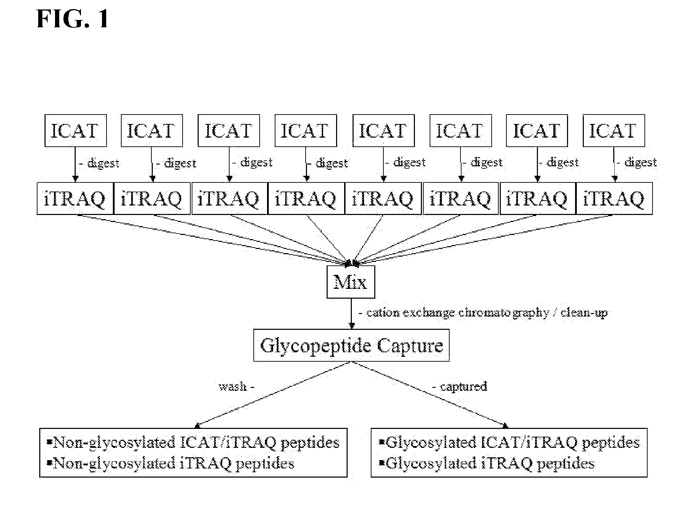

Fig. 1 depicts a schematic illustration of a preferred embodiment of the invention for the selective enrichment of glycosylated peptides (glyco-peptides). First, the proteins comprised in a given sample are isotopically labeled using the Isotope-coded Affinity Tag technology (ICAT) and enzymatically digested. Individual pools of the resulting peptides are then isobarically labeled using the Isobaric Tag for Relative and Absolute Quantitation technology (iTRAQ). The labeled glycosylated peptides are combined and captured via cation exchange chromatography. Finally, the glycosylated ICAT/iTRAQ peptides and glycosylated iTRAQ peptides are separated from their non-glycosylated counterparts.

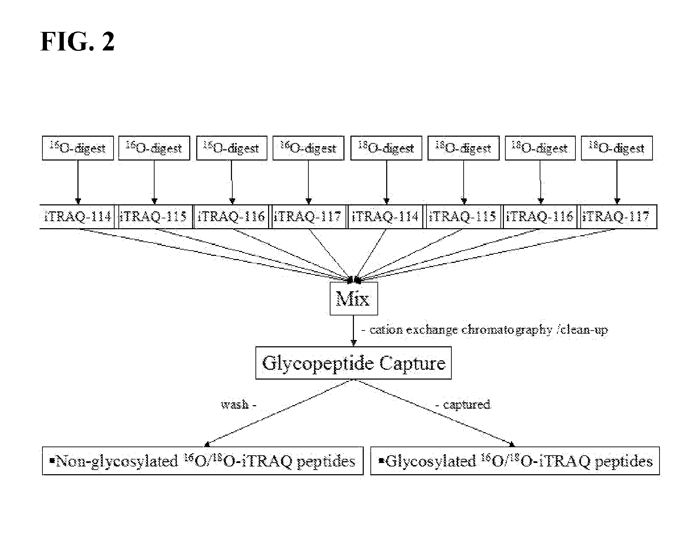

Fig. 2 depicts a schematic illustration of another preferred embodiment of the invention for the selective enrichment of glycosylated peptides. The proteins comprised in a sample are enzymatically digested in the presence of either ¹⁶O- or ¹⁸O-labeled water (isotopic labeling). Individual pools of the resulting peptides are then isobarically labeled using iTRAQ. The labeled glycosylated peptides are combined and captured via cation exchange chromatography. Finally, the glycosylated ¹⁶O/¹⁸O-labeled/iTRAQ peptides are separated from their non-glycosylated counterparts.

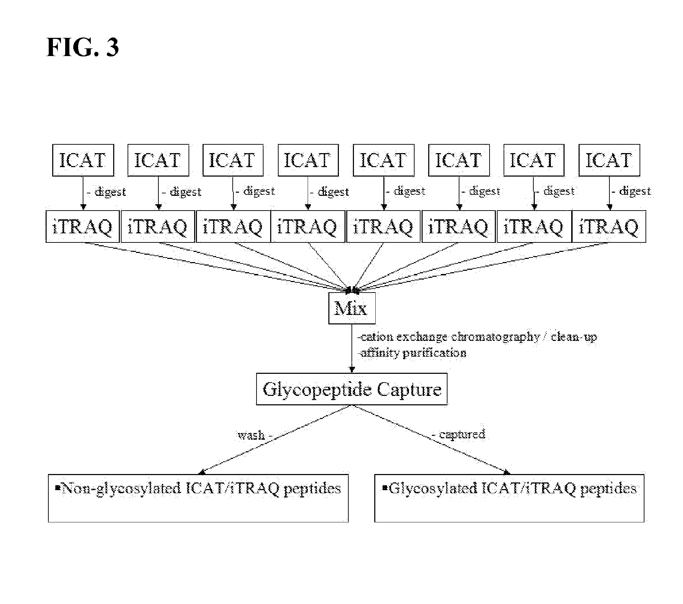

Fig. 3 depicts a schematic illustration of a further preferred embodiment of the invention for the selective enrichment of glycosylated peptides. The proteins are subjected to the same labeling protocol as described in Fig. 1 as well as a two-fold capturing/selection procedure involving affinity selection of the ICAT-peptides (i.e. the cysteine-containing peptides) and a cation exchange chromatography as described in Figs. 1 and 2. Then, the glycosylated ICAT/iTRAQ peptides are separated from their non-glycosylated counterparts.

### DETAILED DESCRIPTION OF THE INVENTION

The present invention is based on the unexpected finding that combining a particular protein/peptide protocol with the specific selection of the post-translationally modified proteins and/or peptides to be analyzed allows the rapid and highly selective enrichment and/or separation of said modified proteins from a complex sample. Furthermore, by adapting the reaction conditions the same method is also appropriate to discriminate between different subsets of proteins bearing a particular post-translational modification.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims.

The drawings described are only schematic and are non-limiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

In one embodiment, the present invention relates to a method for the selective enrichment and/or separation of post-translationally modified proteins and/or peptides from a sample, comprising:
(a) single or double chemical labeling of the proteins and/or peptides comprised in the sample;
(b) capturing a subset of post-translationally modified proteins and/or peptides comprising a specific post-translational modification to be analyzed; and
(c) separating the captured subset of post-translationally modified proteins and/or peptides.

In preferred embodiments of the inventive method, the post-translational modification to be analyzed is glycosylation.

The term "proteins", as used herein, refers to any naturally occurring or synthetic (e.g., generated by chemical synthesis or recombinant DNA technology) macromolecules comprising a plurality of natural or modified amino acids connected via peptide bonds. The length of such a molecules may vary from two to several thousand amino acids (the term thus also includes what is generally referred to as oligopeptides).

Typically, the term "proteins" relates to molecules having a length of more than 20 amino acids. Thus, proteins to be analyzed in the present invention may have a length from about 30 to about 2500 amino acids, from about 50 to about 1000 amino acids or from about 100 to about 1000 amino acids.

The term "peptide", as used herein, refers to any fragments of the above "proteins" that are obtained after cleavage of one or more peptide bonds. A peptide as used in the present invention is not limited in any way with regard to its size or nature. Typically, peptides to be analyzed in the present invention may have a length from about 2 to about 20 amino acids, from about 3 to about 18 amino acids or from about 5 to about 15 amino acids.

The term "post-translational modification", as used herein, denotes any type of chemical modification of a protein and/or peptide according to the invention that takes place after completion of protein translation. Examples of such modifications include *inter alia* phosphorylation, ubiquitinylation, acetylation, glycosylation, alkylation, isoprenylation, and lipoylation, with glycosylation being particularly preferred. The term is also to be understood not to be limited with regard to the numbers and/of types of post-translational modifications being comprised in a protein and/or peptide. Thus, a given protein may comprise in its sequence two or more glycosylated amino acids or one or more phosphorylated amino acids and one or more glycosylated amino acid residues.

The terms "glycosylated proteins" (herein also referred to as "glyco-proteins") and "glycosylated peptides" (herein also referred to as "glyco-peptides"), as used herein, denote any proteins and/or peptides comprising in their primary sequence one or more glycosylated amino acid residues, wherein the glycosylation may be an *N-*glycosylation, an *O*-glycosylation or a glycosylphosphatidylinisotol-anchoring. The term "*N-*glycosylation" (herein also referred to "*N*-linked glycosylation"), as used herein, denotes the enzyme-directed and site-specific addition of any saccharide moiety (i.e. a carbohydrate or sugar moiety including monosaccharides such as glucose or galactose, disaccharides such as maltose and sucrose and oligo- or polysaccharides) to the amide nitrogen of asparagine amino acid residues, while the term "O-glycosylation" (herein also referred to "O-linked glycosylation"), as used herein, refers to the enzyme-directed and site-specific addition of any saccharide moiety to the hydroxy oxygen of serine or threonine amino acid residues. Finally, the term "glycosylphosphatidylinisotol-anchoring" (herein also referred to "GPI-anchoring"), as used herein, denotes the addition of a hydrophobic phosphatidylinositol group linked through a carbohydrate containing linker (such as glucosamine and mannose linked to a phosphoryl ethanolamine residue) to the C-terminal amino acid of a protein and/or peptide, wherein the two fatty acids within the phosphatidylinositol group anchor the protein to the cell membrane. Within the scope of the present invention, amino acid glycosylation may occur *in vivo* by post-translational protein modification or *in vitro* by employing specific glycosyl transferases.

The glyco-proteins and/or -peptides are enriched and/or separated by means of the inventive method from a sample comprising such molecules, preferably from a biological sample. The term "sample", as used herein, is not intended to necessarily include or exclude any processing steps prior to the performing of the methods of the invention. The samples can be unprocessed ("crude") samples, extracted protein fractions, purified protein fractions and the like. For example, the samples employed may be pre-processed by immunodepletion of one or more subsets of abundant proteins. Suitable samples include samples of prokaryotic (e.g., bacterial, viral samples) or eukaryotic origin (e.g., fungal, yeast, plant, invertebrate, mammalian and particularly human samples).

The term "complex sample", as used herein, denotes the fact that a sample analyzed using a method of the present invention typically includes a multitude of different proteins and/or peptides (or different variants of such proteins and/or peptides) present in different concentrations. For example, complex samples within the present invention may include at least about 500, at least about 1000, at least about 5000 or at least about 10000 proteins and/or peptides. Typical complex samples used in the invention include *inter alia* cell extracts or lysates of prokaryotic or eukaryotic origin as well as human or non-human body fluids such as whole blood, serum, plasma samples or the like.

The term "chemical labeling", as used herein, denotes the attachment or incorporation of one or more detectable markers (or "labels") into a protein and/or peptide used in the invention. The term "detectable marker", as used herein, refers to any compound that comprises one or more appropriate chemical substances or enzymes, which directly or indirectly generate a detectable compound or signal in a chemical, physical or enzymatic reaction. As used herein, the term is to be understood to include both the labels as such (i.e. the compound or moiety bound to the protein and/or peptide) as well as the labeling reagent (i.e. the compound or moiety prior to the binding with the peptide or protein). A label used in the present invention may be attached to an amino acid residue of a protein and/or peptide via a covalent or a non-covalent linkage. Typically, the linkage is a covalent linkage. The labels can be selected *inter alia* from isotopic labels, isobaric labels, enzyme labels, colored labels, fluorescent labels, chromogenic labels, luminescent labels, radioactive labels, haptens, biotin, metal complexes, metals, and colloidal gold, with isotonic labels and isobaric labels being particularly preferred. All these types of labels are well established in the art.

The term "single labeling", as used herein, denotes that a protein and/or peptide is labeled with one or more detectable markers of only one type of labels, for example, only isobaric labels. The term "double labeling", as used herein, denotes that a protein and/or peptide is labeled with one or more detectable markers of two different types of labels, for example, isotopic labels and isobaric labels.

In preferred embodiments of the inventive method, the proteins and/or peptides are double labeled. Particularly preferably, the double labeling of the proteins and/or peptides comprises an isotopic labeling and an isobaric labeling, that is the attachment or incorporation of one or more each of isotopic labels and isobaric labels to the proteins and/or peptides to be analyzed, respectively. Within the scope of the present invention, the two labeling steps can be performed in any sequential order or concomitantly. However, in typical embodiments of the inventive method, isotopic labeling precedes the isobaric labeling. Stable labels can be introduced in proteins and/or peptides at various stages of sample preparation, for example by metabolic labeling of growing cells (e.g., using the commercially available SILAC (stable isotope labeling with amino acids in cell culture) technology), labeling of intact proteins (e.g., ICAT labeling), protein digestion in the presence of a label (e.g., ¹⁶O- or ¹⁸O-labeled water), and labeling of digested peptides (e.g., iTRAQ labeling).

The term "isotopic labeling", as used herein, refers to a labeling event using a set of two or more labels having the same chemical formula but differing from each other in the number and/or type of isotopes present of one or more atoms, resulting in a difference in mass of the proteins and/or peptides labeled that can be detected, for example, via mass spectrometry. In other words, otherwise identical proteins and/or peptides labeled with different isotopic labels can be differentiated as such based on difference in mass. While isobaric labels (see below) in principle constitute a specific type of isotopic labels, in the context of the present invention, the term isotopic label will be used to refer to labels which are not isobaric, but can as such be differentiated based on their molecular weight.

Examples of isotopic labels according to the invention include *inter alia* ¹⁶O- or ¹⁸O-labeled water or Isotope-Coded Affinity Tag (ICAT) labels (cf. Gygi, S.P. et al. (1999) Nat. Biotechnol. 17, 994-999). The ICAT reagent uses three functional elements: a thiol-reactive group for the selective labeling of reduced cysteine amino acid residues, a biotin affinity tag to allow for selective isolation of labeled peptides, and an isotopic tag, which is synthesized in two isotopic forms, the "light" (non-isotopic) and the "heavy" (utilizing, for example, ²H or ¹³C) form. Within the scope of the present invention, isotopic labeling may be performed on the peptide level (e.g., by cleaving the proteins comprised in the sample to be analyzed in the presence of either ¹⁶O- or ¹⁸O-labeled water) or directly on the protein level (i.e. prior to cleavage), for example by employing commercially available ICAT reagents (Applied Biosystems, Foster City, CA, USA).

The term "isobaric labeling", as used herein, refers to a labeling event using a set of two or more labels having the same structure and the same mass, which upon fragmentation release particular fragments having - due to a differential distribution of isotopes within the isobaric labels - the same structure but differ in mass. Isobaric labels typically comprise a reporter group which in mass spectrometric analyses generates a strong signature ion upon collision induced dissociation (CID, i.e. a fragment release) and a balance group which comprises a certain compensating number of isotopes so as to ensure that the combined mass of the reporter group and balance group is constant for the different isobaric labels. The balance group may or may not be released from the label upon CID.

Examples of isobaric labels according to the invention include *inter alia* Isobaric Tag for Relative and Absolute Quantitation (iTRAQ) labels (cf. Ross, P.L. et al. (2004) Mol. Cell. Proteomics 3, 1154-1169). This approach employs four different iTRAQ reagents, each containing a reporter group, a balance group and a peptide reactive group which reacts with primary amine groups (for example, the ε amino-group of lysine amino acid residues). The reporter group has a mass of 114, 115, 116 or 117 Da, depending on differential isotopic combinations of ¹²C/¹³C and ¹⁶O/¹⁸O in each reagent. The balance group varies in mass from 31 to 28 Da to ensure that the combined mass of the reporter group and the balance group remains constant (145 Da) for the four reagents. Accordingly, labeling of the same peptide with each of these reagents results in peptides which are isobaric and thus co-elute, for example, in liquid chromatography and consequently are chromatographically indistinguishable from each other. During mass spectrometry, however, at least the respective reporter groups are released upon CID, displaying distinct masses of 114 to 117 Da. The intensity of these fragments can be used for quantification of the individual proteins and/or peptides in a single.

The present invention particularly relates to the combined use of isotopic labels and isobaric labels for multiplexed protein analysis, that is for performing multiple analyses in parallel, for example 2, 4, 8 or 16 parallel samples. In particular, such a combined labeling strategy also enables the comparison of relative protein levels between different samples. The combination of isotopic and isobaric labeling may have the advantage that only those peptides need to be specifically analyzed, for example by MALDI-MS/MS analysis or iTRAQ quantification, for which a differential expression level is observed, thus resulting in a faster and less complex sample analysis.

In preferred embodiments, the method further comprises cleaving the proteins into peptides prior to or concomitant with labeling the proteins and/or peptides. In some embodiments, protein cleavage is performed after the isotopic labeling of the proteins (but prior to an isobaric labeling). Such cleaving of proteins may either be achieved chemically (e.g., via acid or base treatment employing chemicals such as cyanogen bromide, 2-(2'-nitrophenylsulfonyl)-3-methyl-3-bromo-indolenine (BNPS), formic acid, hydroxylamine, iodobenzoic acid, and 2-nitro-5-thiocyanobenzoid acid) or enzymatically via proteases (including *inter alia* trypsin, pepsin, thrombin, papain, and proteinase K) well known in the art.

The term "capturing", as used herein, denotes any procedure for the identification and subsequent enrichment and/or selection of a particular subset of post-translationally modified proteins and/or peptides comprising a specific post-translational modification to be analyzed by means of covalently or non-covalently attaching (and thus immobilizing) said subset of post-translationally modified proteins and/or peptides to a suitable binding member (for example, an appropriate matrix or resin; cf. below) which the proteins and/which allows for further separation of the captured post-translationally modified proteins and/or peptides from their unlabeled counterparts. In preferred embodiments, the subset of post-translationally modified proteins and/or peptides to be captured comprises glycosylated proteins and/or peptides. Optionally, the binding member may be attached to a solid support such as a surface, for example the surface of a paramagnetic polystyrene particle or a latex bead, said immobilization facilitates subsequent separation of the captured subset of proteins and/or peptides.

Typically, the capturing step comprises at least one affinity purification or affinity chromatography step, that is, the attachment (i.e. capturing) of the subset of post-translationally modified proteins and/or peptides to a binding member having specific binding activity for the subset of proteins and/or peptides to be selected. However, the capturing step may also may rely on one or more of ion exchange chromatography, size exclusion chromatography, hydrophobic interaction chromatography and/or reversed-phase chromatography. Within the scope of the present invention, it is also possible to combine two or more capturing steps of the same or of different types, for example two affinity chromatography steps (either using the same type of matrix or different types) or an affinity purification step and a ion exchange chromatography.

In preferred embodiments relating to the analysis of glycosylated proteins and/or peptides the capturing step comprises at least one of lectin affinity capture and glycoprotein chemical capture. The term "lectin affinity capture", as used herein, denotes any capturing protocol employing lectins as a binding member. The term "lectins", as used herein, refers to a class of proteins found in plants, bacteria, fungi, and animals that are known to bind specific oligosaccharide moieties (reviewed, e.g., in Lis, H., and Sharon, N. (1998) Chem. Rev. 98, 637-674). Unlike antigen-antibody binding affinities, the affinity constants for the binding of monosaccharides and oligosaccharides to most lectins are in the low micromolar range but can be in the millimolar range. For affinity capture purposes, it is the multivalent nature of both the oligosaccharides and the lectins themselves that make these interactions useful for chromatography separations. Examples of suitable lectins include *inter alia* α-sarcin, rizin, concavalin A, and calnexin. Several protocols for lectin affinity capture are known in the art (cf, e.g., Kaji, H. et al. (2003) Nat. Biotechnol. 21, 667-672; Hirabayashi, J. (2004) Glycoconj. J. 21, 35-40; Drake, R.R. et al. (2006) Mol. Cell. Proteomics 5, 1957-1967).

The term "glycoprotein chemical capture", as used herein, refer to any chemical capture procedures for glycoproteins notinvolving the use of lectins. Many of these procedures involve an ion exchange chromatography step. Several protocols are well established in the art (cf., e.g., Zhang, H. et al. (2003) Nat. Biotechnol. 21, 660-666; Sun, B. et al. (2007) Mol. Cell. Proteomics 6, 141-149).

Typically, in these chemical capture procedures, the proteins to be analyzed are single or double chemical labeled and cleaved into peptides, for example by using trypsin or any other proteases. The digested peptides were dissolved in a coupling buffer (100 mM sodium acetate, 150 mM NaCl, pH 5.5) at a final concentration of 2 mg/100 µl buffer. Any non-dissolved solids are removed by centrifugation. The supernatant is used for the following reactions. The *cis*-diol groups of the carbohydrates are first oxidized into aldehydes by adding 10 mM sodium periodate (final concentration) and incubating the sample in the dark at room temperature for 30 minutes with end-over-end rotation. Next, 20 mM sodium sulphite (final concentration) are added for quenching and the sample is incubated for 10 min at room temperature to deactivate any excess oxidant.

The coupling reaction is then initiated by introducing a hydrazide resin (in form of beads that are commercially available) at a final concentration of 20 mg/ml into the quenched sample. The aldehyde groups of the carbohydrates are coupled to the hydrazine resin by forming covalent hydrazone bonds. In order to ensure a solid to liquid ratio of 1:5 an appropriate amount of coupling buffer is added to the sample. The coupling reaction is performed at 37 °C overnight with end-over-end rotation.

Subsequently, the resin is washed twice thoroughly and sequentially with milliQ-purified water, 1.5 M NaCl, methanol, and acetonitrile, respectively. Washing was followed by a buffer exchange step (i.e. a cation exchange chromatography step) to adjust a final concentration of 100 mM NH₄HCO₃.

Finally, the captured post-translationally modified proteins and/or peptides (i.e. attached to the binding member and optionally any solid support) are separated from the sample, for example by centrifugation or by magnetic separation, in case magnetic beads are employed.

In preferred embodiments of the invention, the separation step comprises removing the post-translational modification from at least a first subset of the separated post-translationally modified proteins and/or peptides, which facilitated further separation and also allows discrimination between different subsets of the separated post-translationally modified proteins and/or peptides. In particularly preferred embodiments, the post-translational modification to be removed is a glycosylation.

The term "at least a first subset of the separated post-translationally modified proteins and/or peptides", as used herein, is to be understood in such a way that it may relate to the totality of the separated post-translationally modified proteins and/or peptides present or to a particular part thereof.

The term "removing", as used herein, refers to the complete elimination of the post-translational modification to be analyzed, for example by chemical cleavage or enzyme action (see also the discussion below). Thus, removing the post-translational modification from at least a subset of the separated post-translationally modified proteins and/or peptides also results in their release from the binding member (and optionally from the solid support).

Preferably, the post-translational modification is removed chemically (for example, via β-elimination) or enzymatically by means of particular glycosidases.

In one embodiment of the inventive method relating to the selective enrichment and/or separation of glyco-proteins and/or -peptides the at least first subset of the separated glyco-proteins and/or -peptides comprises *N*-glycosylated proteins and/or peptides.

Removal of an *N*-linked gycosyl modification from the proteins and/or peptides may be accomplished by enzymatic cleavage of the *N*-linked peptides from the glycosyl moiety at 37 °C overnight using peptide:*N*-glycosidase F (PNGase F) at a concentration of 500 U (1 µl) PNGase F per 2-6 mg of crude proteins. PNGase F is an amidase that cleaves between the innermost GlcNAc and asparagine residues of high mannose, hybrid, and complex oligosaccharides from *N*-linked glycol-proteins. The supernatant containing the released de-glycosylated peptides can be collected by centrifugation. Thus, this procedure allows for the selective discrimination of *N-*glycosylated proteins and/or peptides from the other types of glyco-proteins and/or - peptides (i.e. *O*-glycosylated and GPI-anchored proteins and/or peptides, respectively).

Although PNGase F deglycosylation removes the sugar moiety from the glyco-peptide, the glycosylation site can still be detected by mass spectrometry analysis, since PNGase F deglycosylation results in an aspartic acid for every asparagines. (corresponding to a mass difference of + 1 Da).

In another typical embodiment, the inventive method, particularly the separation step, further comprises removing the post-translational modification from at least a second subset of the separated post-translationally modified proteins and/or peptides. In one embodiment of the inventive method relating to the selective enrichment and/or separation of glyco-proteins and/or -peptides the at least second subset of the separated glyco-proteins and/or -peptides comprises *O*-glycosylated proteins and/or peptides.

Removal of an *O*-linked gycosyl modification from the proteins and/or peptides may be accomplished by enzymatic cleavage employing particular *O*-glycosidases or chemically such as via a β-elimination (i.e., a type of elimination reaction well established in the art, wherein atoms or atom groups are removed from two adjacent atoms of the substrate while forming a π bond).

In other embodiments, the method further comprises analyzing the separated post-translationally modified proteins and/or peptides by means of mass spectrometry, an analytical technique used to measure the mass-to-charge ratio of ions.. The particular mass spectrometric analysis applied may depend on the levels of protein and/or peptide expression determined in different samples. In some embodiments, the method of the invention are performed in a high-throughput format.

In a further embodiment, the invention relates to the use of a method, as described herein, for performing qualitative and/or quantitative proteomic analyses.

While the above invention has been described with respect to some of its preferred embodiments, this is in no way to limit the scope of the invention. The person skilled in the art is clearly aware of further embodiments and alterations to the previously described embodiments that are still within the scope of the present invention.

### EXAMPLES

### Example 1

The isotopic and isobaric labeling of the proteins comprised in the sample to be analyzed was performed using the commercially available reagents ICAT and iTRAQ, respectively, following the instructions of the manufacturers. After performing the ICAT labeling the proteins were enzymatically cleaved into peptides before adding the iTRAQ reagents. Alternatively, the isotopic labeling step was performed by labeling half of the sample via protease mediated ¹⁶O- or ¹⁸O-incorporation into the C-terminus of peptides present in the sample.

Subsequently, the double labeled peptides were subjected to the glycol-peptide capture procedure. The dried tryptic peptides were dissolved in a coupling buffer (100 mM sodium acetate, 150 mM NaCl, pH 5.5) at a final concentration of 2 mg/100 µl buffer. Any non-dissolved solids were removed by centrifugation. The supernatant was used for the following reactions.

The *cis*-diol groups of the carbohydrates were first oxidized into aldehydes by adding 10 mM sodium periodate (final concentration) and incubating the sample in the dark at room temperature for 30 minutes with end-over-end rotation. Next, 20 mM sodium sulphite (final concentration) were added and the sample was incubated for 10 min at room temperature to deactivate any excess oxidant in the sample.

The coupling reaction was initiated by introducing a commercially available hydrazide resin (beads) at a final concentration of 20 mg/ml into the quenched sample. In order to ensure a solid to liquid ratio of 1:5 an appropriate amount of coupling buffer was added to the sample. The coupling reaction was performed at 37 °C overnight with end-over-end rotation. Subsequently, the resin was washed twice thoroughly and sequentially with milliQ-purified water, 1.5 M NaCl, methanol, and acetonitrile, respectively. Washing was followed by a buffer exchange step (i.e. a cation exchange chromatography step) to adjust a final concentration of 100 mM NH₄HCO₃.

Enzymatic cleavage of the *N*-linked peptides from the glycosyl moiety is carried out at 37 °C overnight using peptide:*N*-glycosidase F (PNGase F) at a concentration of 500 U (1 µl ) PNGase F per 2-6 mg of crude proteins. PNGase F is an amidase that cleaves between the innermost GlcNAc and asparagine residues of high mannose, hybrid, and complex oligosaccharides from *N*-linked glycol-proteins. The supernatant containing the released de-glycosylated peptides was collected by centrifugation and combined with the supernatant of an 80% acetonitrile wash.

Afterwards, the solution was dried, reconstituted with 1% acetonitrile in 0.1% formic acid and subjected to mass spectrometry (MS) analysis. This procedure only selected N-linked glycol-peptides. Although PNGase F deglycosylation removes the sugar moiety from the glyco-peptide, the glycosylation site can still be detected by mass spectrometry analysis, since PNGase F deglycosylation results in a an aspartic acid for every asparagines. Alternatively, *O*-linked glycopeptides can be selectively cleaved by means of a particular *O*-glycosidase or by a chemical cleavage such as a β-elimination.

## Claims

1. Method for the selective enrichment and/or separation of post-translationally modified proteins and/or peptides from a sample, comprising:
(a) single or double chemical labeling of the proteins and/or peptides comprised in the sample;
(b) capturing a subset of post-translationally modified proteins and/or peptides comprising a specific post-translational modification to be analyzed; and
(c) separating the captured subset of post-translationally modified proteins and/or peptides.

2. The method of claim 1, wherein the post-translational modification to be analyzed is glycosylation.

3. The method of claim 1 or 2, further comprising: cleaving the proteins into peptides prior to and/or concomitantly with performing step (a).

4. The method of any of claims 1 to 3, wherein the double labeling comprises an isotopic and an isobaric labeling.

5. The method of claim 4, wherein the isotopic labeling is performed prior to the isobaric labeling.

6. The method of claim 4 or 5, wherein the isotopic labeling is performed prior to cleaving the proteins into peptides.

7. The method of any of claims 2 to 6, wherein step (b) comprises at least one of lectin affinity capture and glycoprotein chemical capture.

8. The method of any of claims 1 to 6, wherein step (c) comprises removing the post-translational modification from at least a first subset of the separated post-translationally modified proteins and/or peptides.

9. The method of claim 8, wherein the post-translational modification is removed chemically or enzymatically.

10. The method of any of claims 1 to 9, further comprising: analyzing the separated proteins and/or -peptides by means of mass spectrometry.

11. The method of any of claims 1 to 10, wherein the method is performed in a high-throughput format.

12. Use of a method of any of claims 1 to 11 for performing qualitative and/or quantitative proteomic analyses.
